# EUROPEAN PATENT APPLICATION

(11) **EP 4 372 073 A1**
(43) Date of publication of application: **22.05.2024**
(21) Application number: 22841218.5
(22) Date of filing: 04.07.2022
(51) Int. Cl.: C12M 1/12, C12M 1/26, C12M 1/36, C12Q 1/24

(54) **QUANTITATIVE, STERILE AND DISPOSABLE AUTOMATIC CELL SAMPLING DEVICE AND METHOD**

(30) Priority: 13.07.2021 CN 202110788193
(71) Applicant: Shenzhen Cellbri Bio-Innovation Technology Co., Ltd., Shenzhen, Guangdong 518107 (CN)
(72) Inventor: GUO, Xiaoliang, Shenzhen, Guangdong 518107 (CN); YAO, Jialin, Shenzhen, Guangdong 518107 (CN); SHANG, Yuanfang, Shenzhen, Guangdong 518107 (CN); ZHENG, Weiwu, Shenzhen, Guangdong 518107 (CN)
(74) Representative: Herrero & Asociados, S.L.
(86) International application number: PCT/CN2022/103738
(87) International publication number: WO 2023/284580

(57) **Abstract**

The present disclosure relates to a quantitative, sterile and disposable automatic cell sampling device and method. The automatic cell sampling device includes a sampling container, a pump, a sterile filter, a control valve, a first conduit, and a second conduit. A receiving cavity is formed in an interior of the sampling container, and a first interface and a second interface are formed in a top portion of the sampling container for achieving a maximum sampling capacity of the sampling container. One end of the first conduit is connected to the first interface, and the other end thereof is connected to a cell based drug production device through the pump. The control valve is installed in a middle of the first conduit. The second conduit is connected between the second interface and the sterile filter, and is configured for discharging and supplementing sterile air in the sampling container. The sterile filter is used to filter the air into sterile air. The automatic cell sampling device has a small volume and realizes a high space utilization of the sampling container and a sterile and convenient sampling, greatly reducing the probability of cell contamination.

## Description

### TECHNICAL FIELD

The present disclosure relates to the field of drug sampling technology, and more particularly, to a quantitative, sterile and disposable automatic cell sampling device and method.

### BACKGROUND

Due to the characteristics of the production process of cell based drugs, such as multiple preparation steps and complex processes, the complex process and the in vitro environment have a significant impact on the biological activities of the cells. Therefore, in the production process of the cell based drugs, regular cell sampling is required to monitor indicators such as a cell activity, a loss rate, and a density. At present when experimental personnel carries out the sampling, sterile sampling devices are often used. The existing sterile sampling method generally involves placing the sampling device into a sterile environment and opening a cover of the sampling device for sampling.

However, the operation of the existing sampling device is troublesome, and frequent opening of the cover can easily result in contamination. In addition, if a container has a large volume, there are higher requirements for handling the container and the sterile environment area, thus, the existing sampling device have the disadvantages of inconvenient sampling and easy contamination.

### SUMMARY OF THE DISCLOSURE

The present disclosure provides a quantitative, sterile and disposable automatic cell sampling device and method. The automatic cell sampling device has a small volume and realizes a high space utilization of a sampling container and a sterile and convenient sampling, greatly reducing the probability of cell contamination.

To achieve the above objectives, the present disclosure provides a quantitative, sterile and disposable automatic cell sampling device, including a sampling container, a pump, a sterile filter, a control valve, a first conduit, and a second conduit;
a receiving cavity is formed in an interior of the sampling container for receiving a sample, and a first interface and a second interface are formed in a top portion of the sampling container communicating with the receiving cavity for achieving a maximum sampling capacity of the sampling container;
one end of the first conduit is connected to the first interface, and the other end of the first conduit being connected to a cell based drug production device through the pump;
the control valve is installed in a middle of the first conduit for controlling on and off of the first conduit;
the second conduit is connected between the second interface and the sterile filter for discharging and supplementing sterile air in the sampling container;
the sterile filter is configured to filter air passing therethrough into the sterile air;
the control valve has an open state and a closed state, wherein when the control valve is in the open state, a communication is formed between two ends of the first conduit; when the control valve is in the closed state, the communication between the two ends of the first conduit are interrupted.

In an embodiment, the pump is a peristaltic pump.

In an embodiment, the sampling container is a transparent container provided with a scale.

In an embodiment, the control valve is a tube clamp, a solenoid valve, an electric valve, a pneumatic valve or a hydraulic valve.

In an embodiment, the sampling container is made of flexible material.

The present disclosure further provides a cell sampling method using the above automatic cell sampling device, wherein cell fluid is introduced into the sampling container by generating a fluid driving force through the pump, and the cell sampling method includes:
closing the control valve of the automatic cell sampling device such that the control valve is in the closed state, connecting the pump of the automatic cell sampling device to the cell based drug production device, and allowing both the first interface and the second interface to be located at highest positions of the sampling container;
opening the control valve and the pump of the automatic cell sampling device, and quantitatively transferring the cell fluid from the cell based drug production device to the receiving cavity of the sampling container through the pump;
closing the control valve and the pump of the automatic cell sampling device, and sealing the first conduit between the sampling container and the control valve to achieve a quantitative, sterile and disposable cell sampling.

In an embodiment, the cell sampling method further includes following steps between the opening the control valve and the pump of the automatic cell sampling device and the closing the control valve and the pump of the automatic cell sampling device:
controlling the pump to rotate reversely, allowing the air in the sampling container and the air filtered by the sterile filter to be introduced into the first conduit, and allowing the cell fluid in the first conduit to flow back into the cell based drug production device.

In an embodiment, in the sealing the first conduit between the sampling container and the control valve, a hot-melt device is used to perform a hot-melt sealing process on the first conduit.

In an embodiment, after the sealing the first conduit between the sampling container and the control valve, the method further includes:
sterilely connecting another automatic cell sampling device to the sealed first conduit, and repeating the above steps to achieve multiple times of sterile sampling.

In an embodiment, in the sterilely connecting another automatic cell sampling device to the sealed first conduit, a first conduit of another automatic cell sampling device is connected to the sealed first conduit through a sterile conduit adapter.

Compared with the prior art, the present disclosure has the following beneficial effects:
The sampling container of the automatic cell sampling device of the present disclosure is connected to the cell based drug production device through the pump, which provides a power source to introduce the cell fluid in the cell based drug production device into the sampling container, enabling a quantitative, sterile and disposable cell sampling. The first and second interfaces of the sampling container are located at the top portion of the sampling container, allowing for the maximum sampling capacity of the cell fluid in the sampling container and achieving a maximum utilization efficiency in the sampling container of a small volume. The control valve is installed in the middle of the first conduit connecting the pump and the sampling container, allowing for easy control of the on and off of a sampling channel, such that the sampling is easy and convenient. In addition, the sampling container is connected to the sterile filter, such that the air entering the sampling container can be filtered through the sterile filter. In order to avoid the waste of cell fluid in the first conduit, the pump can be operated reversely, which can introduce the air in the sampling container and/or the sterile air filtered by the sterile filter into the first conduit, allowing the cell fluid in the first conduit to flow back to the cell based drug production device. Since the sterile filter can filter contamination sources in the working environment, the sterile air can be provided for the sampling container and the contamination of the cell fluid can be avoided. Therefore, the automatic cell sampling device has a small volume and realizes a high space utilization of the sampling container and a sterile and convenient sampling, greatly reducing the probability of cell contamination.

In addition, with the automatic cell sampling device and the cell sampling method of the present disclosure, the function of multiple times of sterile sampling can be realized by connecting multiple automatic cell sampling devices in the cell based drug production device. The multiple automatic cell sampling devices can be connected by configuring multiple interfaces at the first conduit through sterile conduit adapters, or, the automatic cell sampling device can be repeatedly connected through the sterile conduit adapter, which can also achieve the function of multiple times of sterile sampling.

### BRIEF DESCRIPTION OF THE DRAWINGS

FIG. 1 is a schematic diagram showing an automatic cell sampling device in accordance with an embodiment of the present disclosure;
FIG. 2 is a schematic diagram showing an automatic cell sampling device in accordance with another embodiment of the present disclosure; and
FIG. 3 is a process flow diagram of a cell sampling method in accordance with an embodiment of the present disclosure.

Reference signs are as follows:
- 1: Sampling container
- 2: Pump
- 3: Sterile filter
- 4: Control valve
- 5: First conduit
- 6: Second conduit
- 11: First interface
- 12: Second interface

### PREFERRED EMBODIMENTS

The technical solutions in the embodiments of the present disclosure will be clearly and completely described below in conjunction with the accompanying drawings. It is apparent that the described embodiments are only a part of the embodiments of the present disclosure, not all of them. Based on the embodiments of the present disclosure, all other embodiments obtained by those skilled in the art without creative work fall within the scope of protection of the present disclosure.

### Example 1

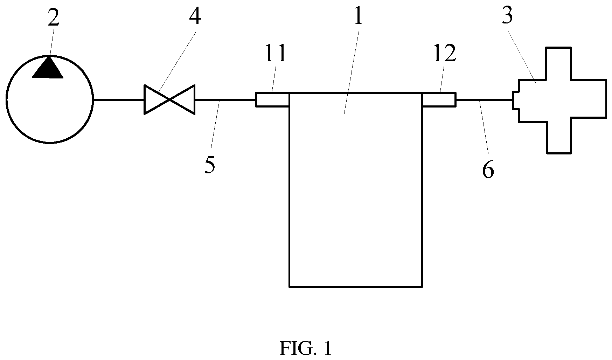

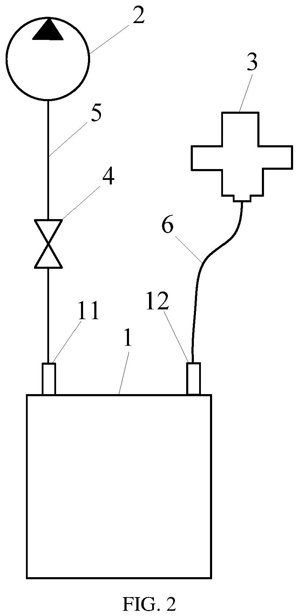

As shown in FIG. 1 and FIG. 2, an embodiment of the present disclosure provides a quantitative, sterile and disposable automatic cell sampling device, which includes a sampling container 1, a pump 2, a sterile filter 3, a control valve 4, a first conduit 5, and a second conduit 6.

A receiving cavity is formed in an interior of the sampling container 1 (not shown) for receiving cell fluid and other samples. A first interface 11 and a second interface 12 are formed in a top portion of the sampling container 1. The first interface 11 and the second interface 12 communicate with the receiving cavity for realizing a maximum sampling capacity of the sampling container 1. In the structure shown in FIG. 1, the first interface 11 is located on a top of a left side of the sampling container 1, and the second interface 12 is located on a top of a right side of the sampling container 1 and is opposite to the first interface 11. In the structure shown in FIG. 2, the first interface 11 is located on a left side of a top surface of the sampling container 1, and the second interface 12 is located on a right side of the top surface of the sampling container 1. During sampling, the first interface 11 is used as an inlet for the cell fluid, and the second interface 12 is used as an outlet for air. When the cell fluid in the first conduit 5 is allowed to flow back to a cell based drug production device, the first interface 11 is used as an outlet for sterile air, and the second interface 12 is used as an inlet for the sterile air filtered by the sterile filter 3. The sampling container 1 can be made of flexible materials such as plastic and rubber or made of rigid material such as plastic and glass. When the sampling container 1 is pressed, the air inside the sampling container 1 is introduced to the cell based drug production device; when the sampling container 1 is released, the cell fluid in the cell based drug production device is sucked into the sampling container 1 due to a negative pressure in the sampling container 1. At this time, a sterile sampling can be achieved without the pump 2.

The pump 2 is configured to drive the cell fluid to flow. The pump 2 can be a peristaltic pump. The pump 2 provides a power source for the sampling, such that the cell fluid can enter the sampling container 1 from the cell based drug production device.

One end of the first conduit 5 is connected to the first interface 11, and the other end of the first conduit 5 is connected to the cell based drug production device through the pump 2. The control valve 4 for controlling an on and off of the first conduit 5 is installed in a middle of the first conduit 5. The first conduit 5 introduces the cell fluid driven by the pump 2 into the sampling container 1. The control valve 4 is configured to control the on and off of the first conduit 5, thus, whether the cell fluid can enter the sampling container 1 or flow back to the cell based drug production device can be controlled through the first conduit 5.

The second conduit 6 is connected between the second interface 12 and the sterile filter 3, and is configured to discharge and supplement the sterile air in the sampling container 1. The second conduit 6 is connected between the sampling container 1 and the sterile filter 3; during the sampling, the sterile air in the sampling container 1 can be discharged through the sterile filter 3; when the cell fluid flows back to the cell based drug production device to avoid a waste of the cell fluid, the second conduit 6 is configured to introduce the sterile air filtered by the sterile filter 3 into the sampling container 1.

The sterile filter 3 is configured to filter the air passing therethrough into sterile air. The sterile filter 3 can sterilely filter the air to prevent external contamination sources from entering the sampling container 1 or the cell based drug production device through the sterile filter 3. The sterile filter 3 can be provided with a cover.

The control valve 4 has an open state and a closed state. When the control valve 4 is in the open state, two ends of the first conduit 5 are in a communicating state; when the control valve 4 is in the closed state, the two ends of the first conduit 5 are in a disconnected state. The control valve 4 can be a manual control valve 4 or an automatic control valve 4, such as a pipe clamp, a solenoid valve, an electric valve, a pneumatic valve, and a hydraulic valve.

The sampling container 1 of the automatic cell sampling device described above is connected to the cell based drug production device through the pump 2, and the cell fluid in the cell based drug production device is introduced to the sampling container 1 by the power source provided by the pump 2, enabling a quantitative, sterile and disposable cell sampling. The first interface 11 and the second interface 12 are arranged on the top portion of the sampling container 1, enabling the maximum sampling capacity of the cell fluid in the sampling container 1, achieving a maximum cell fluid capacity in the sampling container 1 of a small volume, and improving an utilization efficiency of the sampling container 1. The control valve 4 is installed in the middle of the first conduit 5 connecting the pump 2 and the sampling container 1, allowing easy control of the on and off of the sampling channel through the control valve 4, and providing an easy and convenient sampling.

Since the sampling container 1 is connected to the sterile filter 3 through the second conduit 6, the air entering the sampling container 1 can be filtered by the sterile filter 3 to prevent contamination sources from entering the sampling container 1 and the cell based drug production device. In order to avoid the waste of cell fluid in the first conduit 5, the pump 2 can be operated reversely, which can introduce the air in the sampling container 1 and/or the sterile air filtered by the sterile filter 3 into the first conduit 5, such that the cell fluid in the first conduit 5 can flow back to the cell based drug production device. Since the sterile filter 3 can filter the contamination sources in the working environment and provide sterile air for the sampling container 1, the contamination of the cell fluid is avoided.

Therefore, the automatic cell sampling device of the present disclosure has a small volume and realizes a high space utilization of the sampling container 1 and a sterile and convenient sampling, which greatly reduces the probability of cell contamination.

In order to facilitate precise control of the sampling volume, the sampling container 1 can be a transparent container with a scale. With the transparent container with the scale, the sampling volume of the cell fluid can be visually observed, and the precise sampling volume of the cell fluid can be directly obtained.

### Example 2

The present disclosure provides a cell sampling method using the above automatic cell sampling device, in which the fluid driving force generated by the pump 2 is used to introduce the cell fluid into the sampling container 1. The cell sampling method includes steps as follows.

Step S10, closing the control valve 4 of the automatic cell sampling device such that the control valve 4 in the closed state, connecting the first conduit 5 of the automatic cell sampling device to the cell based drug production device, and allowing the first interface 11 and the second interface 12 to be located at highest positions of the sampling container 1.

Step S20, opening the control valve 4 and the pump 2 of the automatic cell sampling device, and transferring the cell fluid of the cell based drug production device quantitatively to the receiving cavity of the sampling container 1 through the pump 2, and opening the control valve 4 and the pump 2 of the automatic cell sampling device to allow the control valve 4 to be in the open state and the pump 2 to be in a working state.

Step S40, closing the control valve 4 and the pump 2 of the automatic cell sampling device, sealing the first conduit 5 between the sampling container 1 and the control valve 4 to achieve a quantitative, sterile and disposable cell sampling, and closing the control valve 4 and the pump 2 of the automatic cell sampling device, that is, allowing the control valve 4 to be in the closed state and the pump 2 to stop working.

In the above-mentioned cell sampling method, the pump can provide the power source required for the sampling, which allows for convenient and rapid sampling from the cell based drug production devices, and achieves the functions of quantitative, sterile, and disposable cell sampling.

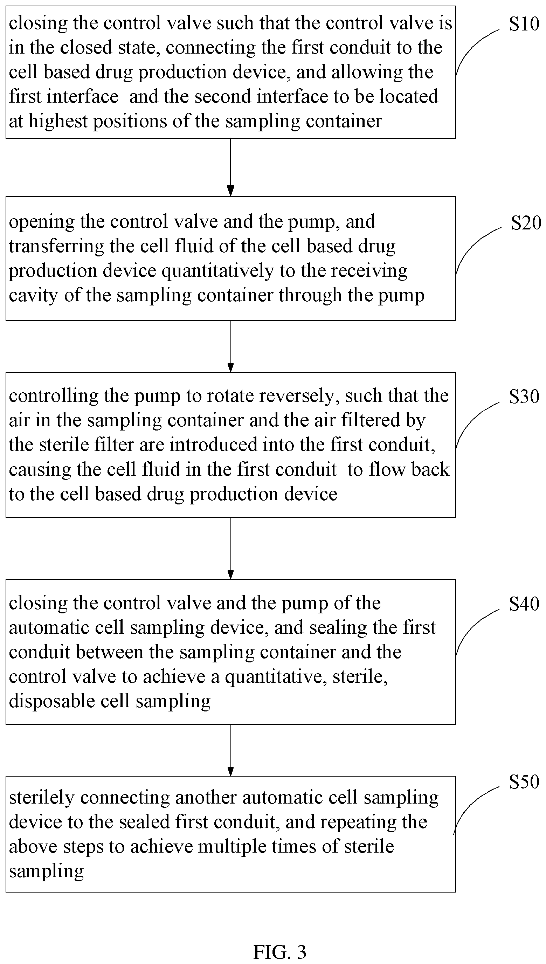

As shown in FIG. 3, the present disclosure also provides a cell sampling method using the above automatic cell sampling device in accordance with another embodiment. The cell fluid is introduced into the sampling container 1 by the fluid driving force generated by the pump 2. The cell sampling method includes steps as follows.

Step S10, closing the control valve 4 of the automatic cell sampling device to allow the control valve 4 to be in the closed state, connecting the first conduit 5 of the automatic cell sampling device to the cell based drug production device, and allowing the first interface 11 and the second interface 12 both to be located at highest positions of the sampling container 1.

Step S20, opening the control valve 4 and the pump 2 of the automatic cell sampling device, quantitatively transferring the cell fluid of the cell based drug production device to the receiving cavity of the sampling container 1 through the pump 2, and opening the control valve 4 and the pump 2 of the automatic cell sampling device, that is, allowing the control valve 4 to be in the open state and the pump 2 to be in a working state.

Step S30, controlling the pump 2 to rotate reversely, such that the air in the sampling container 1 and the air filtered by the sterile filter 3 are introduced into the first conduit 5, causing the cell fluid in the first conduit 5 to flow back to the cell based drug production device.

Step S40, closing the control valve 4 and the pump 2 of the automatic cell sampling device, and sealing the first conduit 5 between the sampling container 1 and the control valve 4 to achieve a quantitative, sterile and disposable cell sampling, and closing the control valve 4 and the pump 2 of the automatic cell sampling device, that is, allowing the control valve 4 to be in the closed state and the pump 2 to stop working.

In the above-mentioned cell sampling method, the pump can provide the power source required for the sampling, which not only facilitates and speeds up the sampling from cell based drug production devices, achieves the functions of quantitative, sterile and disposable cell sampling, but also enables the cell fluid in the first conduit 5 to flow back to the cell based drug production device under sterile conditions through the reverse rotation of the pump 2, avoiding the waste of the cell fluid in the first conduit 5.

As shown in FIG. 3, in order to achieve multiple times of sterile sampling, based on the various cell sampling methods described above, the cell sampling method may further include the following steps after sealing the first conduit 5 between the sampling container 1 and the control valve 4: step S50, sterilely connecting another automatic cell sampling device to the sealed first conduit 5, and repeating the above steps to achieve multiple times of sterile sampling. That is, after a cell fluid sampling is completed using an automatic cell sampling device, another sterile cell sampling device can be connected to the sealed first conduit 5, and the steps S10, S20, and S40 can be repeated, or the steps S10, S20, S30, and S40 can be repeated to complete the second cell fluid sampling. And the whole process can be repeated to achieve the function of multiple times of cell fluid sampling.

By sterilely connecting another automatic cell sampling device to the sealed first conduit 5, the above steps S10-S40 can be repeated to achieve the second sterile sampling. By repeating steps S50, S10, S20, S40, or steps S50, S10, S20, S30, S40, multiple times of sterile sampling can be achieved, allowing for convenient and rapid multiple times of sterile sampling without contamination, thus improving the flexibility of the sterile sampling.

In order to achieve the sterile connection between the sealed first conduit 5 and another automatic cell sampling device, the first conduit 5 of the another automatic cell sampling device can be connected to the first conduit 5 using a sterile conduit adapter.

Based on the above cell sampling method, using a hot-melt device to perform a hot-melt sealing process on the first conduit 5 between the sampling container 1 and the control valve 4 can facilitate and quickly achieve a disposable cell sampling of cell fluid.

Obviously, those skilled in the art can make various modifications and variations to the embodiments of the present disclosure without departing from the spirit and scope of the invention. Therefore, if these modifications and variations of the present disclosure fall within the scope of the claims of the present disclosure and their equivalent technologies, the present disclosure is also intended to include these modifications and variations.

## Claims

1. A quantitative, sterile and disposable automatic cell sampling device, comprising a sampling container, a pump, a sterile filter, a control valve, a first conduit, and a second conduit;
a receiving cavity being formed in an interior of the sampling container for receiving a sample, and a first interface and a second interface being formed in a top portion of the sampling container communicating with the receiving cavity for achieving a maximum sampling capacity of the sampling container;
one end of the first conduit being connected to the first interface, and the other end of the first conduit being connected to a cell based drug production device through the pump;
the control valve being installed in a middle of the first conduit for controlling on and off of the first conduit;
the second conduit being connected between the second interface and the sterile filter for discharging and supplementing sterile air in the sampling container;
the sterile filter being configured to filter air passing therethrough into the sterile air;
the control valve having an open state and a closed state, wherein when the control valve is in the open state, a communication is formed between two ends of the first conduit; when the control valve is in the closed state, the communication between the two ends of the first conduit is interrupted.

2. The automatic cell sampling device according to claim 1, wherein the pump is a peristaltic pump.

3. The automatic cell sampling device according to claim 1, wherein the sampling container is a transparent container provided with a scale.

4. The automatic cell sampling device according to any one of claims 1 to 3, wherein the control valve is a tube clamp, a solenoid valve, an electric valve, a pneumatic valve or a hydraulic valve.

5. The automatic cell sampling device according to any one of claims 1 to 3, wherein the sampling container is made of flexible material.

6. A cell sampling method using the automatic cell sampling device according to any one of claims 1 to 5, wherein cell fluid is introduced into the sampling container by generating a fluid driving force through the pump, and the cell sampling method comprises:
closing the control valve of the automatic cell sampling device such that the control valve is in the closed state, connecting the pump of the automatic cell sampling device to the cell based drug production device, and allowing both the first interface and the second interface to be located at highest positions of the sampling container;
opening the control valve and the pump of the automatic cell sampling device, and quantitatively transferring the cell fluid from the cell based drug production device to the receiving cavity of the sampling container through the pump;
closing the control valve and the pump of the automatic cell sampling device, and sealing the first conduit between the sampling container and the control valve to achieve a quantitative, sterile and disposable cell sampling.

7. The cell sampling method according to claim 6, further comprising following steps between the opening the control valve and the pump of the automatic cell sampling device and the closing the control valve and the pump of the automatic cell sampling device:
controlling the pump to rotate reversely, allowing the air in the sampling container and the air filtered by the sterile filter to be introduced into the first conduit, and allowing the cell fluid in the first conduit to flow back into the cell based drug production device.

8. The cell sampling method according to claim 6, wherein in the sealing the first conduit between the sampling container and the control valve, a hot-melt device is used to perform a hot-melt sealing process on the first conduit.

9. The cell sampling method according to any one of claims 6 to 8, wherein after the sealing the first conduit between the sampling container and the control valve, the method further comprises:
sterilely connecting another automatic cell sampling device to the sealed first conduit, and repeating the above steps to achieve multiple times of sterile sampling.

10. The cell sampling method according to claim 9, wherein in the sterilely connecting another automatic cell sampling device to the sealed first conduit, a first conduit of another automatic cell sampling device is connected to the sealed first conduit through a sterile conduit adapter.
